# EUROPEAN PATENT APPLICATION

(11) **EP 4 174 225 A1**
(43) Date of publication of application: **03.05.2023**
(21) Application number: 21832106.5
(22) Date of filing: 12.01.2021
(51) Int. Cl.: C40B 60/14, C12M 1/00

(54) **THREE-DEGREE-OF-FREEDOM LIBRARY PREPARATION CARTRIDGE AND METHOD**

(30) Priority: 29.06.2020 CN 202010605311
(71) Applicant: 3D Biomedicine Science & Technology Co., Limited, Shanghai 201114 (CN)
(72) Inventor: WANG KENG MENG, Albert, Shanghai 201114 (CN); QIN, Zi, Shanghai 201114 (CN)
(74) Representative: Weidner Stern Jeschke
(86) International application number: PCT/CN2021/071165
(87) International publication number: WO 2022/001084

(57) **Abstract**

The present invention relates to a three-degree-of-freedom library preparation cassette and a method using the same. Two-degree-of-freedom movement capability is provided for mutual positioning between a pipette and reagent wells, within a limited small volume, by combining rotation of a mixing reagent tray and relative horizontal movement between the pipette and the mixing reagent tray, thus greatly increasing the number of reagent wells that can be provided, thereby increasing the functions and applications of the whole system; and the device is simple in structure, low in cost, and suitable for disposable use.

## Description

### Field of the Invention

The present invention relates to the technical field of gene sequencing devices, and in particular to a three-degree-of-freedom library preparation cassette and a method using the same.

### Background of the Invention

After the 1950s, with the development of molecular genetics, people gradually realized that the nature of gene is DNA fragments that control biological characters and have genetic effects. On the one hand, genes can faithfully replicate themselves to ensure basic characteristics of organisms; on the other hand, genes may mutate, resulting in diseases. Gene sequencing can rapidly and accurately acquire biological genetic information, and reveal the complexity and diversity of a genome, and plays a very important role in life science research; similarly, gene sequencing is also of great significance and value for clinical medicine, and the mechanism research and clinical diagnosis of many diseases gradually rely on gene sequencing technology.

Library preparation is one of essential and important steps in gene sequencing work. In traditional library preparation by manual operations, the accomplishment of such processes as sample addition, warm bath reaction, purification and elution requires human participation, and the whole process is carried out in a relatively open laboratory environment. Operating in this way have the disadvantages that manual operations have a high probability of errors and lead to uncontrollable results, and also, samples are very liable to cause environmental contamination, and the risk of cross-contamination between the samples is greatly increased. Therefore, automated, closed library preparation devices and methods represent a major direction of research.

Invention patent application No. CN201080031828.3, entitled "Apparatus for Performing Amplicon Rescue Multiplex PCR", discloses a cassette structure for PCR amplification and DNA amplicon detection, wherein pipetting is performed between a plurality of chambers arranged in a straight line on a base by a suction tube capable of moving vertically and horizontally within a sealed cassette; invention patent application No. CN201580007560.2, entitled "Composite Liquid Cell (CLC) Mediated Nucleic Acid Library Preparation Device, and Methods for Using the Same", discloses a library preparation device comprising a robotically controlled pipette moving correspondingly between 6 plate locations; invention application No. CN201611190715.3, entitled "Full-Automatic RNA Library Preparation Device for use in Second-Generation High-Throughput Sequencing", discloses a full-automatic RNA library preparation device comprising 9 reagent bins arranged sequentially in a straight line and a sampling probe that moves under the control of a screw; invention patent plication No. CN201910106150.3, entitled "Gene Sequencing Library Preparation Device", discloses an external device for library preparation capable of driving an injection unit to ascend and descend and a round cassette base plate to rotate, wherein corresponding operations can be performed on a closed cassette by using the external device; invention patent application No. CN201910106163.0, entitled "Closed Sequencing Library Preparation Cassette", discloses a closed cassette for library preparation, and especially for application to the preparation device described in the above-mentioned invention patent application No. CN201910106150.3, provides an injection unit capable of moving vertically up and down and a round base plate capable of rotating, wherein the injection unit can, by rotation of the round base plate, perform pipetting operations corresponding sequentially to multiple accommodating slots in a circle concentrically arranged circumferentially on the round base plate. All of the above prior art proposed closed, automated solutions for the library preparation process, but there are still problems in practical applications to library preparation.

Firstly, in the above prior art, a library preparation device as a whole mostly adopts a two-degree-of-freedom movement structure, and at least one of two degrees of freedom needs to be used for vertical movement of a pipette (suction pipette, sampling probe, etc., which are different expressions of the same functional component), i.e., only one degree of freedom is left for mutual positioning movement between the pipette and a reagent well (chamber, reagent bin, accommodating slot, etc., which are different expressions of the same functional component), and regardless of whether this degree of freedom originates from horizontal movement of the pipette or single-degree-of-freedom movement of the reagent well component, it greatly limits the number of reagent wells that can be effectively contained and used in a limited volume. Secondly, the use of a closed library preparation device has a major advantage that it reduces harsh requirements on the operating environment for library preparation, i.e., library preparation operations can be performed at any location free from the confines of a high-grade laboratory. In particular, a small-size device can support operations in environments that support rapid deployment, such as vehicle-mounted platforms, which greatly expands the application scope of library preparation. The technical solution as disclosed in application No. CN201580007560.2 supports up to hundreds of reagent wells, but the volume of the device as a whole is huge, and in fact, it merely integrates manual operation steps that would have been carried out in an open environment into a closed housing, and the operations are directly carried out by a robot instead of a human, However, the operation process of the device involves replacing a carrying plate and other operations that needs to open the housing of the device, so it still needs to be arranged in a fixed laboratory environment that meets requirements. Finally, the library preparation process requires high precision and is very sensitive to contamination, so equipment and devices that directly contact reagents in the operations must be disposable, which also limits the library preparation device itself from using an overly complex structure to reduce loss and waste.

The three problems presented above are essentially interrelated. The paradox is that the library preparation process is diverse and complex, so there is a need to provide as many reagent wells as possible to meet different library preparation needs and improve operational efficiency; however, the requirements for a small volume and disposable use limit the number of reagent wells that can be provided by the library preparation device, such that the functional needs of library preparation cannot be met.

### Summary of the Invention

To address the shortcomings of the prior art, the present invention proposes a three-degree-of-freedom library preparation cassette and a method that, in addition to one degree of freedom for vertical movement of a pipette, provide two degrees of freedom for mutual positioning movement between the pipette and a reagent well, within a limited small volume, thus greatly increasing the number of reagent wells that can be provided; and the device is simple in structure, low in cost, and suitable for disposable use.

To achieve the above object, the present invention adopts the following technical solution:
Athree-degree-of-freedom library preparation cassette, including a pipette and a mixing reagent tray arranged within the sealed cassette, wherein
the pipette is at least vertically movably arranged within the sealed cassette;
the mixing reagent tray is a circular tray and is rotatable clockwise or counterclockwise at least about a geometric center thereof as an axis; the mixing reagent tray is provided with a plurality of reagent wells, which are configured to at least include two different well pitches;
a downward projection relative straight-line distance between the pipette and the geometric center of the mixing reagent tray is changeable by horizontal movement of the pipette or the mixing reagent tray, so that the pipette corresponds vertically to any reagent well on the mixing reagent tray as selected and performs a liquid drawing or liquid injection operation on the reagent well; and
the well pitches comprise a straight-line distance from the geometric center of the mixing reagent tray to a geometric center of the reagent well, and/or a straight-line distance from the geometric center of the mixing reagent tray to a position on the reagent well corresponding to the liquid drawing or liquid injection operation by the pipette.

Further, the pipette moves horizontally from the geometric center of the mixing reagent tray to an edge of the mixing reagent tray along any straight line passing through the geometric center of the mixing reagent tray.

Further, the mixing reagent tray is provided with a plurality of reagent wells arranged annularly successively in concentric circles with three different well pitches from inside to outside, forming three groups consisting of a plurality of reagent wells arranged successively along the concentric circles of different well pitches, respectively, namely, an inner circle reagent well group, a middle circle reagent well group and an outer circle reagent well group.

Further, the inner circle reagent well group comprises at least one magnetic bead reagent well, which is detachable from the mixing reagent tray; the middle circle reagent well group comprises a plurality of library construction reagent wells; and the outer circle reagent well group at least comprises a plurality of reaction reagent wells, a plurality of library capture reagent wells, one cleaning reagent well, and one waste reagent well.

Further, the magnetic bead reagent well, the library construction reagent wells, reaction reagent well, the library capture reagent wells, the cleaning reagent well and the waste reagent well have different volumes and/or shapes.

Further, at least one of the plurality of reaction reagent wells is detachable from the mixing reagent tray.

Further, the sum of downward projected areas of the magnetic bead reagent well, the library construction reagent wells, the reaction reagent wells, the library capture reagent wells, the cleaning reagent well and the waste reagent well occupies 90% or more of a downward projected area of the mixing reagent tray.

Further, the mixing reagent tray is further integrally formed with a driven gear, and an external driving device drives, through gear engaged connection, the driven gear to rotate and cause the mixing reagent tray to rotate.

Further, the driven gear does not occupy a top reagent well region of the mixing reagent tray.

Further, the mixing reagent tray is further provided with a limit strip, which is controlled to lock/release the rotation of the mixing reagent tray.

Further, the pipette is connected to a rotating shaft and a screw, respectively, ends of which extend out of the sealed cassette; the screw rotates and drives the pipette to move horizontally; and the rotating shaft rotates and causes a vertical driving gear provided on the rotating shaft to rotate and drive a pipette head component of the pipette to move vertically.

Further, the rotating shaft is in clearance fit with the vertical driving gear provided on the rotating shaft, and the vertical driving gear is capable of freely moving axially along the rotating shaft; and the rotating shaft and the vertical driving gear are in circumferential limit connection having at least one tangential surface to transmit torque.

Further, the ends of the rotating shaft and the screw extending out of the sealed cassette are each provided with both an inner spline and an outer spline; the external driving device transmits torque to the rotating shaft and the screw by combined action of an outer spline and an inner spline corresponding to the inner spline and the outer spline.
Further, the cassette further includes a mixing reagent tray base, a pipette pump assembly and a cassette housing;
the mixing reagent tray base includes a bottom seat and a top cover; the mixing reagent tray is placed within the mixing reagent tray base and is provided with vertical movement limits and horizontal plane movement limits by the bottom seat and the top cover;
the pipette pump assembly provides a liquid drawing or liquid injection driving force for the pipette; and
the cassette housing is independently provided with a hot lid capable of heating and thermally insulating specified reagent wells and a sampling through hole capable of opening/sealing and closing specified reagent wells.

The present invention also relates to a method of using a three-degree-of-freedom library preparation cassette, including the following steps:
S 1, cassette installation: installing a mixing reagent tray containing required reagents sealed therein, into a cassette housing by means of a mixing reagent tray base to form a complete cassette;
S2, library preparation: connecting the complete cassette installed with the mixing reagent tray to an external driving device, and using the external driving device to operate the mixing reagent tray to rotate, and enable horizontal movement of the pipette and vertical movement of the pipette, to accomplish a library preparation process; and
S3, reaction product drawing and waste disposal: disconnecting the used cassette from the external external driving device, and properly disposing the same as a waste.

Further, step S 1 includes mounting a detached separately stored magnetic bead reagent well loaded with magnetic beads onto the mixing reagent tray.

Further, step S 1 includes locking the mixing reagent tray in an initial position of preparation by using a limit strip.

Further, step S2 includes releasing the mixing reagent tray to rotate by pushing the limit strip by the external driving device.

Further, step S2 includes heating and thermally insulating the reaction reagent wells provided in the mixing reagent tray by using a hot lid.

Further, step S3 includes drawing the preparation products from the reaction reagent wells through a sampling through hole.

The present invention has the following beneficial effects:
Using the three-degree-of-freedom library preparation cassette described in the present invention, a three-degree-of-freedom movement structure of a system is implemented ingeniously in a limited volume, thus greatly increasing the number of reagent wells that can be provided by the system in the same volume, thereby increasing the functions and applications of the whole system; while increasing the number of reagent wells, the structural complexity of disposable parts of the system is reduced as much as possible, and the needs for movements in the degrees of freedom can be met by using a simple transmission structure, which achieves a stable transmission effect, and easy and convenient installation and disassembly, and the cassette is especially suitable for disposable use and quick replacement during operations. The present invention also relates to a method of using a three-degree-of-freedom library preparation cassette. By using the method, a variety of library preparation operations can be implemented with high efficiency, and the problem of reagent contamination in open-type operations is avoided.

### Brief Description of the Drawings

Fig. 1 is a schematic diagram of a first embodiment of a three-degree-of-freedom library preparation cassette of the present invention.
Fig. 2 is an exploded diagram of the first embodiment of the three-degree-of-freedom library preparation cassette of the present invention.
Fig. 3 is a schematic diagram of an embodiment of a pipette of the present invention.
Fig. 4 is a top-view diagram of the first embodiment of a mixing reagent tray of the present invention.
Fig. 5 is a schematic diagram of an embodiment of assembly of the mixing reagent tray and a mixing reagent tray base of the present invention.
Fig. 6 is a schematic diagram of an embodiment of assembly of a three-degree-of-freedom library preparation cassette and an external driving device of the present invention.
Fig. 7 is a schematic diagram of distribution of reagent wells in a second embodiment of a mixing reagent tray of the present invention.

Reference numerals: 1 - pipette, 11 - rotating shaft, 12 - screw, 2 - mixing reagent tray, 21 - reagent well, 211 - magnetic bead reagent well, 212 - library construction reagent well, 213 - reaction reagent well, 214 - cleaning reagent well, 215 - waste reagent well, 216 - library capture reagent well, 22 - driven gear, 23 - limit strip, 3 - mixing reagent tray base, 31 - bottom seat, 32 - top cover, 4 - pipette pump assembly, 5 - cassette housing, 51 - hot lid, 52 - sampling through hole, 6 - external driving device.

### Detailed Description of the Embodiments

For clearer understanding of the present invention, it will be described in detail in conjunction with the accompanying drawings and embodiments.

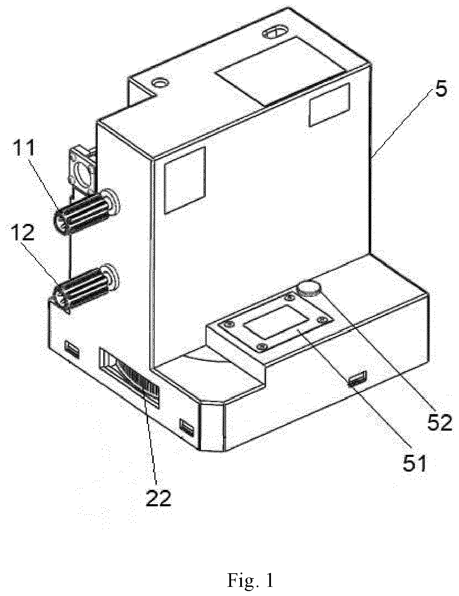

As shown in Fig. 1, which is an overall schematic diagram of the appearance of a first embodiment of a three-degree-of-freedom library preparation cassette of the present invention, it can be seen that most of main components of the cassette are arranged within a sealed cassette housing 5, and parts exposed outside the cassette housing 5 mainly include a rotating shaft 11 and a screw 12 for connecting an external driving device 6, and a driven gear 22, i.e., the cassette as shown in Embodiment 1 has three drive locations independent from each other and thus has the ability to provide three-degree-of-freedom driving. Also visible from the outside of the cassette housing 5 are a hot lid 51 for heating and thermally insulating some specified wells of reagent wells 21 and a sampling through hole 52 for drawing liquid from some specified wells of the reagent wells 21; the sampling through hole 52 is mainly used for drawing prepared library reagents, so the sampling through hole 52 needs to be kept sealed and closed during library preparation and is opened only when samples need to be put before library preparation and when the prepared library reagents, that is the preparation products, need to be carried out after preparation.

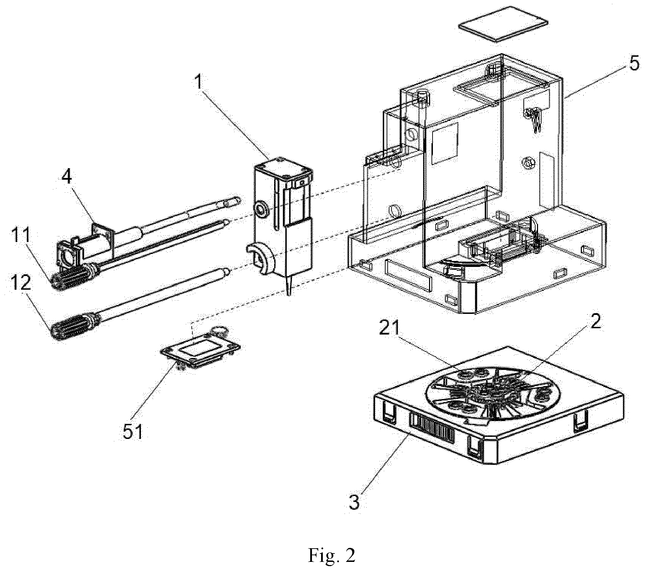

As shown in Fig. 2, which is an exploded view of the first embodiment, the structure of the interior of the cassette in the first embodiment can be seen from Fig. 2. A mixing reagent tray 2 with a number of reagent wells 21 is detachably mounted on the bottom of the cassette housing 5 by being arranged in a mixing reagent tray base 3; a pipette 1 is connected with the rotating shaft 11 and the screw 12 successively, and a pipette pump assembly 4 connected is used to provide a driving force for liquid drawing and injection operations. At a lower end of the pipette 1 is a pipette head component for drawing and injecting liquid. By rotation of the screw 12, the pipette 1 can be controlled to move forward and backward along the screw, and in conjunction with rotation of the mixing reagent tray 2, it enables vertical alignment of the pipette head component of the pipette 1 with any reagent well 21, and then by rotation of the rotating shaft 11 to drive the pipette head component of the pipette 1 to descent vertically, the pipette head component can puncture a thin film on the reagent well 21, extend into the aligned reagent well 21 and come into contact with a reagent stored in the reagent well 21 to perform a drawing operation. A similar operation process is applied to a reagent liquid injection operation.

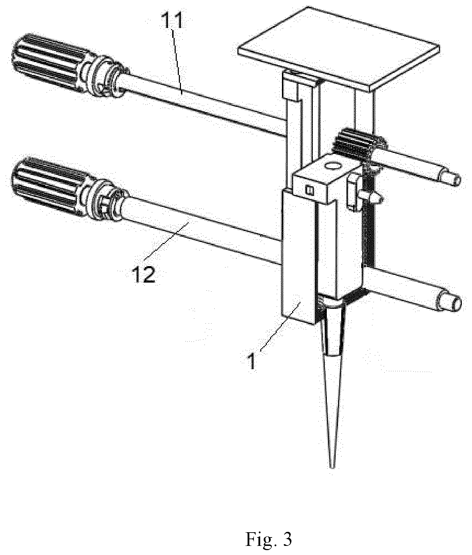

As shown in Fig. 3, which is a schematic structural diagram of an embodiment of the pipette 1, it mainly explains how the pipette 1 described in the present invention is driven by a combination of the rotating shaft 11 and the screw 12 to achieve a horizontal and vertical two-degrees-of-freedom movement capability. The screw 12 responsible for driving the pipette 1 to move horizontally forward and backward is configured as a driving structure of a higher priority, and positive and reverse rotation of the screw 12 can drive the pipette 1 to move horizontally forward and backward overall; the rotating shaft 11 responsible for driving the pipette 1 to move vertically is a driving structure of a lower priority than the screw 12, and positive and reverse rotation of the rotating shaft 11 only drives the pipette head component of the pipette 1 to move vertically up and down; to ensure pipetting precision, the pipette head component of the pipette 1 first descends to the bottom of the well where pipetting is to be performed, the bottom of the well being used as an initial position, then moves up a certain distance, and thereby draws and/or releases the liquid; furthermore, torque transmission of the rotating shaft 11 is implemented by circumferential limit connection including at least one tangential surface, so that the torque transmission of the rotating shaft 11 does not affect the horizontal movement of the pipette 1, and simultaneous horizontal and vertical movements of the pipette 1 can be achieved.

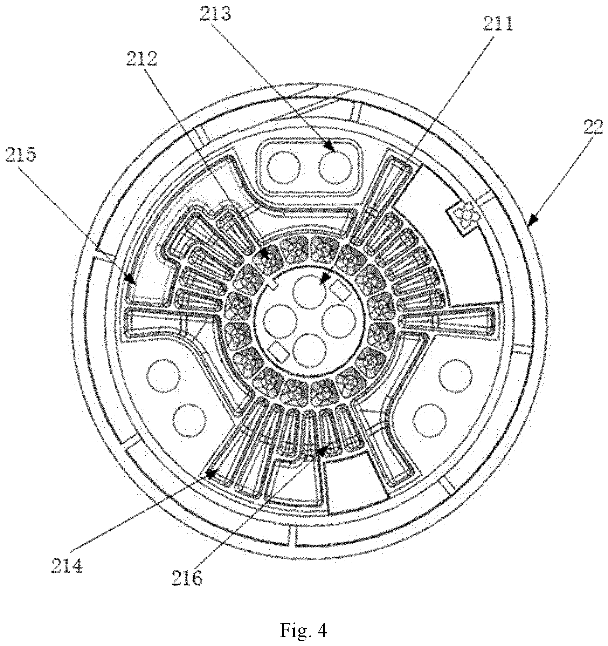

Another focus of the present invention is a design of reagent wells with different well pitches to increase the number of reagent wells 21 that can be accommodated in the mixing reagent tray 2. In traditional library preparation cassette design, reagent wells are mostly arranged in a straight line or in a concentric circle with the same well pitch, which can reduce the structural complexity of pipetting components in the cassette, but also greatly limits the number of reagent wells that can be arranged, because in the case the volume of the cassette is controlled, in order to ensure effective drawing and injection of reagents, the area occupied by the reagent wells cannot be reduced indefinitely. As shown in Fig. 4, which is a schematic diagram of a preferred embodiment of a mixing reagent tray 2 that can be used in the cassette of the present invention, it achieves several times the number of reagent wells 21 in the same volume in the prior art by classifying and arranging the reagent wells 21 with different functions into three concentric circles with different well pitches, and uses a design of a driven gear 22 integrally formed in an outer circle.In this embodiment, an inner circle of the mixing reagent tray 2 is provided with four magnetic bead reagent wells 211, and the four magnetic bead reagent wells 211 can all be loaded with the same magnetic bead reagent to provide a sufficient amount of reagent, and may also be loaded with different magnetic bead reagents respectively as needed so that a single cassette can support the use of multiple types of magnetic bead reagents at the same time, and the section of the magnetic bead reagent wells 211 is designed to be detachable from the mixing reagent tray 2 so that the magnetic bead reagent can be stored separately; from inside to outside, a middle circle of the mixing reagent tray 2 is provided with up to sixteen library construction reagent wells 212 independent from each other, which can be loaded with the same or different reagents as needed, thus greatly increasing functions that can be achieved by the cassette described in the present invention; an outer circle part is independently provided with a total of six reaction reagent wells, every two of which are arranged in a group, respectively, for implementing the main reaction process, wherein one or more groups of reaction reagent wells can be detachable from the mixing reagent tray and can be replaced with other functional modules (e.g. QC modules) as needed, to further increase the use functions of the cassette; the outer circle of the reagent tray 2 is further provided with thirteen library capture reagent wells 216 independent from each other, which are loaded with various reagents used in library capture, thus greatly increasing the functions that can be achieved by the cassette described in the present invention; the remaining part of the outer circle is provided with a plurality of irregular reagent wells 21, including cleaning reagent wells 214 and waste reagent wells 215 indispensable for experiments, the cleaning reagent wells 214 and waste reagent wells 215 making full use of the remaining space on the mixing reagent tray 2, ultimately achieving the effect that more than 90% of the area of the mixing reagent tray 2 is effectively used.

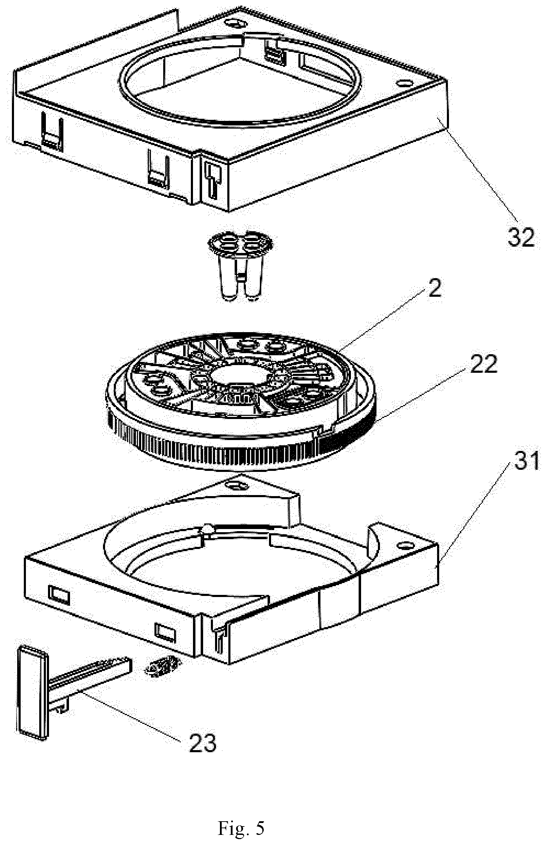

As shown in Fig. 5, which is a schematic diagram of an embodiment of assembly of the mixing reagent tray and the mixing reagent tray base of the present invention, using the mixing reagent tray base 3 can limit vertical and horizontal movements of the mixing reagent tray 2 by a bottom seat 31 and a top cover 32, and enables the mixing reagent tray 2 to be detachably mounted to the cassette housing 5. Furthermore, to facilitate the use of the cassette, the mixing reagent tray 2 is also provided with a limit strip 23 for rotational limit. In the embodiment shown in Fig. 5, the limit strip 23 is a mechanical limit structure, which is inserted into the mixing reagent tray base 3 so that its end limit tooth comes into matched contact with the driven gear 22 to limit rotation of the mixing reagent tray 2. This can simplify the operation process during initialization of the cassette, and facilitates initialization positioning.

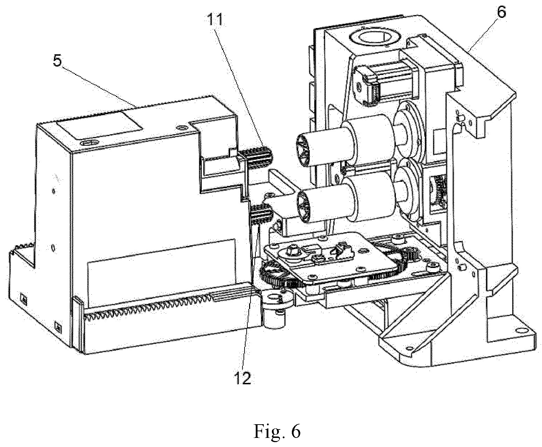

The connection between the cassette as a whole and the external driving device 6 can be as shown in Fig. 6. The rotating shaft 11 and the screw 12 each transmit torque through an inner spline and an outer spline protruding from the cassette housing 5, and the external driving device 6 is provided with corresponding outer splines and inner splines, so that the torque is shared by using matched connections of the two sets of inner and outer splines in the torque transmission process, and the problem of precision reduction caused by device abrasion can be effectively avoided; and the mixing reagent tray 2 is driven by engaged connection of the driven gear 22 and a driving gear on the external driving device 6 to achieve three-degree-of-freedom driving capability of the cassette as a whole. In this configuration, main driving structures are arranged on the external driving device 6 and do not need direct contact with the reagents, and can be used repeatedly; and the cassette part has a simple structure and a low cost, and is suitable for disposable use to avoid reagent contamination.

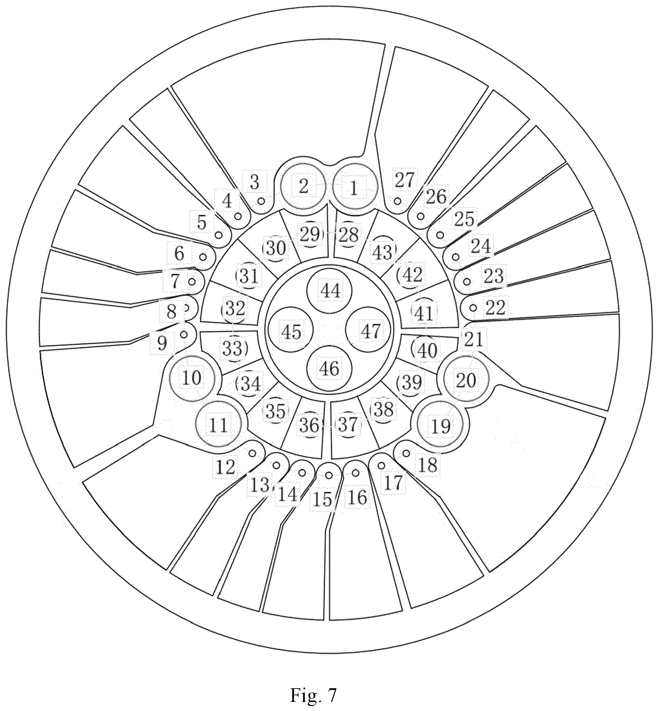

To further illustrate the three-degree-of-freedom library preparation cassette described in the present invention, a typical library construction process is described using a second embodiment of the mixing reagent tray 2 with distribution of the reagent wells 21 as shown in Fig. 7. It is to be noted that all numerical symbols marked in Fig. 7 are independent numbers of different reagent wells 21 thereof, i.e., the mixing reagent tray 2 has 47 mutually independent reagent wells 21 numbered from 1 to 47, as shown in Fig. 7. To avoid confusion, in the following description of the library construction process, specific reagent wells 21 are denoted in the form of w+number, e.g. w1, w6 or w30. Reagent well numbers and corresponding reagent well names/types are provided in Table 1 below.

**Table 1**

| Reagent well number | Reagent well name/type |
|---|---|
| w1 | Reaction well 1 |
| w2 | Reaction well 2 |
| w3 | Waste liquid well |
| w5 | Magnetic bead washing reagent |
| w6 | Magnetic bead mixing reagent |
| w10 | Captured magnetic bead washing reagent 1 |
| w11 | |
| w12 | Magnetic bead washing reagent |
| w19 | Captured magnetic bead washing reagent 2 |
| w20 | Captured magnetic bead washing reagent 2 |
| w21 | Captured magnetic bead washing reagent 1 |
| w22 | Captured magnetic bead washing reagent 1 |
| w23 | Captured magnetic bead washing reagent 3 |
| w25 | Captured magnetic bead washing reagent 4 |
| W26 | Library elution reagent |
| w27 | Magnetic bead washing reagent |
| w28 | End-filling reagent |
| w30 | Connecting reagent 1 |
| w31 | Connecting reagent 2 |
| w32 | PCR amplification reagent 1 |
| w33 | PCR amplification primer 1 |
| w37 | Blocking reagent |
| w38 | Capture Reagent |
| w40 | Capture reagent 2 |
| w42 | PCR amplification reagent 2 |
| w43 | PCR amplification primer 2 |
| w45 | magnetic bead |
| w46 | Captured magnetic bead |
| w47 | magnetic bead |

Library preparation using the mixing reagent tray 2 as shown in Fig. 7 includes the following steps:
a DNA is transferred to w1;
an end-filling reagent is transferred from w28 to w1;
mixing is performed;
a bottom module is rotated;
a PCR module covers the tube bottom;
a hot lid is lowered to cover w1 and w2;
reaction is performed;
reaction is completed;
the bottom module leaves w1;
the hot lid leaves w1 and w2;
a connecting reagent 1 is transferred from w30 to w1;
a connecting reagent 2 is transferred from w31 to w1;
mixing is performed sufficiently;
a heating module is raised;
heated reaction is performed;
reaction is completed;
the heating module is removed;
magnetic beads are mixed;
magnetic beads are transferred from w45 or w47 to w1;
mixing is performed sufficiently;
standing is performed for certain time;
the bottom is rotated, and a magnetic module covers the underside of w1;
standing is performed for certain time;
a supernatant is removed;
a washing reagent is transferred from any of w5, w12 and w27 to w1;
standing is performed for certain time;
a supernatant is removed;
a washing reagent is transferred from any of w5, w12 and w27 to w1;
standing is performed for certain time;
a supernatant is removed;
the magnetic module leaves w1;
a PCR amplification reagent 1 is transferred from w32 to w1;
a PCR amplification primer 1 is transferred from w33 to w1;
mixing is performed sufficiently;
the PCR module covers w1;
the hot lid covers w1 and w2;
PCR reaction is performed;
the bottom PCR module is removed;
the hot lid is removed;
a magnetic bead mixing reagent is transferred from w6 to w1;
mixing is performed sufficiently and evenly;
standing is performed for certain time;
the bottom is rotated, and the magnetic module covers the underside of w1;
standing is performed for certain time;
a supernatant is removed;
a washing reagent is transferred from any of w5, w12 and w27 to w1;
standing is performed for certain time;
a supernatant is removed;
a washing reagent is transferred from any of w5, w12 and w27 to w1;
standing is performed for certain time;
a supernatant is removed;
the magnetic module leaves w1;
an eluent is transferred to w1;
mixing is performed sufficiently and evenly;
standing is performed for certain time;
the bottom is rotated, and the magnetic module covers the underside of w1;
standing is performed for certain time;
a supernatant is drawn from w1 into w2;
library construction is completed;
a cleaning solution is drawn to clean w1;
all liquid in w1 is transferred away;
a blocking solution is added from w37 to w2;
magnetic beads are mixed uniformly;
magnetic beads of a fixed volume are drawn from w45 or w47 into w2;
mixing is performed sufficiently and evenly;;
standing is performed for certain time;
the bottom is rotated, and the magnetic module covers the underside of w2;
standing is performed for certain time;
a supernatant is removed;
a washing reagent is transferred from any of w5, w12 and w27 to w2;
standing is performed for certain time;
a supernatant is removed;
a washing reagent is transferred from any of w5, w12 and w27 to w2;
standing is performed for certain time;
a supernatant is removed;
the magnetic module leaves w2;
a capture reagent is transferred from w38 to w2;
mixing is performed sufficiently and evenly;;
standing is performed for certain time;
the bottom is rotated, and the magnetic module covers the underside of w2;
standing is performed for certain time;
a supernatant is transferred from w2 to w1;
a cleaning solution is drawn to clean w2;
all liquid in w2 is transferred away;
a bottom heating module is raised to cover the underside of w1 and w2;
the hot lid is lowered to cover w1 and w2;
heated reaction is performed;
a side magnetic heating module covers w10 and w11;
a bottom magnetic heating module is raised to cover the underside of w19 and w20;
captured magnetic beads are cleaned;
the captured magnetic beads are mixed;
the capture beads are transferred from w46 to w11;
standing is performed for certain time;
all supernatant in w11 is removed;
a captured magnetic bead cleaning reagent is transferred to w11;
standing is performed for certain time;
all supernatant in w11 is removed;
a captured magnetic bead cleaning reagent is transferred to w11;
standing is performed for certain time;
all supernatant in w11 is removed;
a capture reagent 2 is transferred from w40 to w11;
the hot lid is opened;
all the reagents in w11 are transferred to w2;
mixing is performed sufficiently and evenly;
the hot lid is closed;
standing is performed for certain time;
the hot lid is opened;
a capture washing reagent 1 is drawn from w21 or w22 into w2;
the reagents in w2 are mixed sufficiently and evenly;
the bottom module is replaced with a side magnetic module;
w10 and w11 are interchanged, and w19 and w20 are interchanged, synchronously, and the temperature is maintained;
standing is performed for certain time;
a supernatant is drawn and removed from w2;
a capture washing reagent 2 is drawn from w19 or w20 into w2;
the bottom module is replaced with the heating module;
w10 and w11 are interchanged, and w19 and w20 are interchanged, synchronously, and the temperature is maintained;
mixing is performed sufficiently and evenly;
standing is performed for certain time;
the bottom module is replaced with the side magnetic module;
w10 and w11 are interchanged, and w19 and w20 are interchanged, synchronously, and the temperature is maintained;
a supernatant is drawn and removed from w2;
a capture washing reagent 2 is drawn from w19 or w20 into w2;
the bottom module is replaced with the heating module;
w10 and w11 are interchanged, and w19 and w20 are interchanged, synchronously, and the temperature is maintained;
mixing is performed sufficiently and evenly;
standing is performed for certain time;
the bottom module is replaced with the side magnetic module;
a supernatant is drawn and removed from w2;
all bottom modules are removed;
a magnetic bead washing reagent (room temperature) is drawn from w5 into w2;
mixing is performed sufficiently and evenly;
the side magnetic module covers w2;
standing is performed for certain time;
a supernatant is drawn and removed;
a capture washing reagent 3 is drawn from w23 into w2;
the bottom module is removed;
mixing is performed sufficiently and evenly;
the side magnetic module covers w2;
standing is performed for certain time;
a supernatant is drawn and removed;
a capture washing reagent 4 is drawn from w25 into w2;
the bottom module is removed;
mixing is performed sufficiently and evenly;
the side magnetic module covers w2;
standing is performed for certain time;
a supernatant is drawn and removed;
the bottom module is removed;
a capture PCR amplification reagent 2 is drawn from w42 into w2;
a capture PCR amplification primer 2 is drawn from w43 into w2;
mixing is performed sufficiently and evenly;
the bottom PCR module covers w1 and w2;
the hot lid is lowered to cover w1 and w2;
PCR reaction is performed;
PCR reaction is completed and the hot lid is removed;
the PCR module is removed;
magnetic beads are mixed;
magnetic beads of a certain volume are added from w45 or w47 into w2;
mixing is performed sufficiently and evenly;
standing is performed for certain time;
the side magnetic module covers w2;
standing is performed for certain time;
a supernatant is drawn and removed;
a magnetic bead washing reagent 2 is drawn into w2;
standing is performed for certain time;
a supernatant is drawn and removed;
a magnetic bead washing reagent 2 is drawn into w2;
standing is performed for certain time;
a supernatant is drawn and removed;
a library elution reagent is drawn from w26 into w2;
the bottom module is removed;
mixing is performed sufficiently and evenly;
the side magnetic module is raised;
standing is performed for certain time;
a supernatant is drawn into w1;
the bottom module is removed;
the hot lid covers w1 and w2 to prevent volatilization; and
capture library construction is complete.

The use of the three-degree-of-freedom library preparation cassette described in the present invention can also simplify amplification operation steps in the prior art. Again with reference to the distribution of reagent wells 21 as shown in Fig. 7, reagent well names/types as shown in Table 2 can preferably be used in amplification operations.

**Table 2**

| Reagent well number | Reagent well name/type |
|---|---|
| w1 | Reaction well 1 |
| w2 | Reaction well 2 |
| w3 | PCR1 solution chamber |
| w4 | Primer chamber |
| w5 | Washing solution 1 chamber |
| w6 | Eluent chamber |
| w7 | PCR2 reaction solution chamber |
| w8 | Washing solution 2 chamber |
| w9 | Waste liquid chamber |
| w10 | Recovery chamber |
| w44 | Magnetic bead chamber |

Specific amplification operation steps include:
the position of the cassette is initialized, and it is confirmed that each well is at a correct position;
the cassette is rotated to move to a predetermined position;
a pipetting device is moved to a specified position;
a bottom disc is rotated so that W3 is under a suction pipette;
a transfer pipette punctures a foil seal on w3;
the transfer pipette further goes down into W3 and identifies the bottom position;
the transfer pipette is raised to a predetermined height position;
the disc is rotated so that w1 is at a specified position;
the transfer pipette transfers liquid into w1;
the transfer pipette returns to a predetermined height;
the transfer pipette punctures a foil seal on w4;
the transfer pipette further goes down into w4 and identifies the bottom position;
a certain amount of liquid is drawn;
the transfer pipette is raised to a predetermined height position;
the disc is rotated so that w1 is at a specified position;
the transfer pipette transfers the liquid into w1;
the transfer pipette evenly mixes the liquid in w1 by repeated operations of suction and release;
the transfer pipette returns to a predetermined height;
the hot lid is pressed down to cover the position of w1;
a base PCR unit is raised to cover the underside of w1;
the base unit starts an entire PCR cyclic process;
after the process is completed, the hot lid leaves w1;
a bottom module leaves w1;
the transfer pipette is moved to w44;
a certain volume of magnetic beads is drawn;
the transfer pipette is raised to a predetermined height position;
the transfer pipette is moved to a fixed position above w1;
a fixed volume of magnetic beads is added into w1;
the transfer pipette evenly mixes the liquid in w1 by up and down suction and release;
the transfer pipette leaves w1 to a specified height position;
standing is performed for certain time;
a module with an annular magnetic bracket at the bottom is moved to cover the underside of w1;
standing is performed for certain time;
the transfer pipette enters w1 and draws a certain amount of liquid;
the transfer pipette leaves w1 to a specified height position;
the bottom is rotated so that w9 is placed under the transfer pipette;
the liquid is transferred to w9;
the transfer pipette is moved to a fixed height;
the bottom disc is rotated so that w5 is under the transfer pipette;
the transfer pipette sucks a certain volume of washing solution;
the transfer pipette is moved to a fixed height;
the disc is rotated so that w1 is under the transfer pipette;
the transfer pipette transfers the liquid into w1;
the transfer pipette is moved to a fixed height;
standing is performed for certain time;
the transfer pipette is moved into w1 and draws a certain amount of liquid;
the transfer pipette is moved to a fixed height;
the bottom is rotated so that w9 is placed under the transfer pipette;
the liquid is transferred to w9;
the transfer pipette is moved to a fixed height;
the bottom disc is rotated such that w5 is under the transfer pipette;
the transfer pipette sucks a certain volume of washing solution;
the transfer pipette is moved to a fixed height;
the disc is rotated so that w1 is under the transfer pipette;
the transfer pipette transfers the liquid into w1;
the transfer pipette is moved to a fixed height;
standing is performed for certain time;
the transfer pipette is moved into w1 and draws a certain amount of liquid;
the transfer pipette is moved to a fixed height;
the bottom is rotated so that w9 is placed under the transfer pipette;
the liquids is transferred to w9;
the transfer pipette is moved to a fixed height;
the bottom disc is rotated so that w7 is under the transfer pipette;
the transfer pipette draws a certain volume of reagent;
the transfer pipette is moved to a fixed height;
the bottom module leaves w1;
the bottom disc is rotated so that the position of w1 is under the transfer pipette;
the transfer pipette releases the liquid into w1;
the transfer pipette evenly mixes the liquid in w1 by suction and release;
the transfer pipette is moved to a fixed height;
standing is performed for certain time;
a bottom magnetic module is raised to cover the underside of w1;
standing is performed for certain time;
the transfer pipette sucks a certain volume of liquid;
the transfer pipette is moved to a fixed height;
the bottom disc is rotated so that w2 is under the transfer pipette;
the transfer pipette releases the liquid into w2;
the bottom magnetic module is detached from w2;
the bottom module covers the position of w2;
the hot lid is lowered to cover w2;
reaction is performed with the module;
after the process is complete, the hot lid leaves w2;
the bottom module leaves w2;
the transfer pipette is moved to w44;
a certain volume of magnetic beads is drawn;
a transfer pipette spring rises such that the tip of the transfer pipette leaves w44 to a predetermined height position;
the transfer pipette is moved to a fixed position above w2;
a fixed volume of magnetic beads is added into w2;
the transfer pipette evenly mixes the liquid in w2 by up and down suction and release;
the transfer pipette leaves w2 to a specified height position;
standing is performed for certain time;
the module with the annular magnetic bracket at the bottom is moved to cover the underside of w2;
standing is performed for certain time;
the transfer pipette enters w2 and draws a certain amount of liquid;
the transfer pipette leaves w2 to a specified height position;
the bottom is rotated so that w9 is placed under the transfer pipette;
the liquid is transferred to w9;
the transfer pipette is moved to a fixed height;
the bottom disc is rotated so that w8 is under the transfer pipette;
the transfer pipette sucks a certain volume of washing solution;
the transfer pipette is moved to a fixed height;
the disc is rotated so that w2 is under the transfer pipette;
the transfer pipette transfers the liquid into w2;
the transfer pipette is moved to a fixed height;
standing is performed for certain time;
the transfer pipette is moved into w2 and draws a certain amount of liquid;
the transfer pipette is moved to a fixed height;
the bottom is rotated so that w9 is placed under the transfer pipette;
the liquid is transferred to w9;
the transfer pipette is moved to a fixed height;
the bottom disc is rotated so that w8 is under the transfer pipette;
the transfer pipette sucks a certain volume of washing solution;
the transfer pipette is moved to a fixed height;
the disc is rotated so that w2 is under the transfer pipette;
the transfer pipette transfers the liquid into w2;
the transfer pipette is moved to a fixed height;
standing is performed for certain time;
the transfer pipette is moved into w2 and draws a certain amount of liquid;
the transfer pipette is moved to a fixed height;
the bottom disc is rotated so that w10 is moved to under the transfer pipette;
the liquid is released to w10;
the transfer pipette returns to a specified position;
hot lid is pressed down to over w10; and
the process is completed.

Described above are only preferred specific implementations of the present invention, but the protection scope of the present invention is not limited thereto, and all changes or substitutions that are readily conceivable to those skilled in the art within the technical scope disclosed by the present invention should be encompassed within the protection scope of the present invention. Thus, the protection scope of the present invention should be defined by the claims.

## Claims

1. A three-degree-of-freedom library preparation cassette, comprising a pipette and a mixing reagent tray arranged within the sealed cassette, wherein
the pipette is at least vertically movably arranged within the sealed cassette;
the mixing reagent tray is a circular tray and is rotatable clockwise or counterclockwise at least about a geometric center thereof as an axis; the mixing reagent tray is provided with a plurality of reagent wells, which are configured to at least include two different well pitches;
a downward projection relative straight-line distance between the pipette and the geometric center of the mixing reagent tray is changeable by horizontal movement of the pipette or the mixing reagent tray, so that the pipette corresponds vertically to any reagent well on the mixing reagent tray as selected and performs a liquid drawing or liquid injection operation on the reagent well; and
the well pitches comprise a straight-line distance from the geometric center of the mixing reagent tray to a geometric center of the reagent well, and/or a straight-line distance from the geometric center of the mixing reagent tray to a position on the reagent well corresponding to the liquid drawing or liquid injection operation by the pipette.

2. The cassette according to claim 1, wherein the pipette moves horizontally from the geometric center of the mixing reagent tray to an edge of the mixing reagent tray along any straight line passing through the geometric center of the mixing reagent tray.

3. The cassette according to claim 2, wherein the mixing reagent tray is provided with a plurality of reagent wells arranged annularly successively in concentric circles with three different well pitches from inside to outside, forming three groups consisting of a plurality of reagent wells arranged successively along the concentric circles of different well pitches, respectively, namely, an inner circle reagent well group, a middle circle reagent well group and an outer circle reagent well group.

4. The cassette according to claim 3, wherein the inner circle reagent well group comprises at least one magnetic bead reagent well, which is detachable from the mixing reagent tray; the middle circle reagent well group comprises a plurality of library construction reagent wells; and the outer circle reagent well group at least comprises a plurality of reaction reagent wells, a plurality of library capture reagent wells, one cleaning reagent well, and one waste reagent well.

5. The cassette according to claim 4, wherein the magnetic bead reagent well, the library construction reagent wells, reaction reagent well, the library capture reagent wells, the cleaning reagent well and the waste reagent well have different volumes and/or shapes.

6. The cassette according to claim 4, wherein at least one of the plurality of reaction reagent wells is detachable from the mixing reagent tray.

7. The cassette according to claim 5, wherein the sum of downward projected areas of the magnetic bead reagent well, the library construction reagent wells, the reaction reagent wells, the library capture reagent wells, the cleaning reagent well and the waste reagent well occupies 90% or more of a downward projected area of the mixing reagent tray.

8. The cassette according to claim 2, wherein the mixing reagent tray is further integrally formed with a driven gear, and an external driving device drives, through gear engaged connection, the driven gear to rotate and cause the mixing reagent tray to rotate.

9. The cassette according to claim 8, wherein the driven gear does not occupy a top reagent well region of the mixing reagent tray.

10. The cassette according to claim 9, wherein the mixing reagent tray is further provided with a limit strip, which is controlled to lock/release the rotation of the mixing reagent tray.

11. The cassette according to claim 2, wherein the pipette is connected to a rotating shaft and a screw, respectively, ends of which extend out of the sealed cassette; the screw rotates and drives the pipette to move horizontally; and the rotating shaft rotates and causes a vertical driving gear provided on the rotating shaft to rotate and drive a pipette head component of the pipette to move vertically.

12. The cassette according to claim 11, wherein the rotating shaft is in clearance fit with the vertical driving gear provided on the rotating shaft, and the vertical driving gear is capable of freely moving axially along the rotating shaft; and the rotating shaft and the vertical driving gear are in circumferential limit connection having at least one tangential surface to transmit torque.

13. The cassette according to claim 11, wherein the ends of the rotating shaft and the screw extending out of the sealed cassette are each provided with both an inner spline and an outer spline; the external driving device transmits torque to the rotating shaft and the screw by combined action of an outer spline and an inner spline corresponding to the inner spline and the outer spline.

14. The cassette according to any one of claims 1 to 13, wherein the cassette further comprises a mixing reagent tray base, a pipette pump assembly and a cassette housing;
the mixing reagent tray base comprises a bottom seat and a top cover; the mixing reagent tray is placed within the mixing reagent tray base and is provided with vertical movement limits and horizontal plane movement limits by the bottom seat and the top cover;
the pipette pump assembly provides a liquid drawing or liquid injection driving force for the pipette; and
the cassette housing is independently provided with a hot lid capable of heating and thermally insulating specified reagent wells and a sampling through hole capable of opening/sealing and closing specified reagent wells.

15. A method of using a three-degree-of-freedom library preparation cassette, comprising the following steps:
S1, cassette installation: installing a mixing reagent tray containing required reagents sealed therein, into a cassette housing by means of a mixing reagent tray base to form a complete cassette;
S2, library preparation: connecting the complete cassette installed with the mixing reagent tray to an external driving device, and using the external driving device to operate the mixing reagent tray to rotate, and enable horizontal movement of the pipette and vertical movement of the pipette, to accomplish a library preparation process; and
S3, reaction product drawing and waste disposal: disconnecting the used cassette from the external external driving device, and drawing reaction products from reaction reagent wells, and properly disposing the remaining part as a waste.

16. The method according to claim 15, wherein step S1 comprises mounting a detached separately stored magnetic bead reagent well loaded with magnetic beads onto the mixing reagent tray.

17. The method according to claim 15, wherein step S1 comprises locking the mixing reagent tray in an initial position of preparation by using a limit strip.

18. The method according to claim 17, wherein step S2 comprises releasing the mixing reagent tray to rotate by pushing the limit strip by the external driving device.

19. The method according to claim 15, wherein step S2 comprises heating and thermally insulating the reaction reagent wells provided in the mixing reagent tray by using a hot lid.

20. The method according to claim 15, wherein step S3 comprises drawing the preparation products from the reaction reagent wells through a sampling through hole.
